# EUROPEAN PATENT APPLICATION

(11) **EP 2 123 320 A1**
(43) Date of publication of application: **25.11.2009**
(21) Application number: 08396006.2
(22) Date of filing: 20.05.2008
(51) Int. Cl.: A61M 16/01

(54) **Arrangement and method for supervising medical monitor**

(71) Applicant: GENERAL ELECTRIC COMPANY, Schenectady, NY 12345 (US)
(72) Inventor: Heinonen, Erkki, 00750 Helsinki (FI); Karlsson, Kai, 00660 Helsinki (FI); Weckström, Kurt, 02710 Espoo (FI); Haveri, Heikki, 03150 Huhmari (FI)
(74) Representative: Kanerva, Arto

(57) **Abstract**

An arrangement for supervising a medical monitor is disclosed herein. The arrangement for supervising the medical monitor (1) includes a measurement function (12) including at least one measurement device (13,14,15) to make at least one measurement for obtaining a measurement information for monitoring one or more patient care signal. The arrangement for supervising the medical monitor also includes a supervision analyzer (27) communicating with the measuring function supplying the measurement information to the supervision analyzer and which supervision analyzer is adapted to obtain a reference information created by one of the measurement function measuring patient care signal different from the measurement information or exploiting only partly same measurement components (20-26) while measuring the same patient care signal as measuring the measurement information or other than the measurement function and to compare the measurement information to the reference information to find out whether the measurement function is working properly.

## Description

### BACKGROUND OF THE INVENTION

This disclosure relates generally to an arrangement and method for supervising a medical monitor.

Inhalation anesthesia agents are volatile halogenated hydrocarbon compounds. They are expensive, environmentally harmful, and long-term exposure may be dangerous. Therefore inhalation anesthesia systems are designed to minimize a use of the agents.

In an inhalation anesthesia the volatile agent is vaporized from a liquid form to a vapor mixed in a patient's inhalation gas mixture. From the lungs the anesthesia agent dissolves into a blood that transports the agent to a brain, other organs, muscles, and a fat tissue. Dissolving to tissues increases a body concentration. A concentration in the brain determines a depth of an anesthesia and during an anesthesia all body compartments fill to the concentration needed for an adequate depth of anesthesia. The agents are variable in a solubility to various body compartments, but a relatively slow body metabolism is a common characteristic of those all. Therefore, once the body compartments get filled, body agent uptake is drastically reduced to compensate the small metabolic demand.

To maintain the body agent concentration, an alveolar concentration has to be in equilibrium with the body. To facilitate this without an additional agent consumption, a current delivery system use a re-breathing circuit where an expired breathing gas containing the anesthesia agent in an alveolar concentration is re-circulated back to an inspiration gas. However, before the re-breathing, the expired gas needs to be cleared from the patient-produced CO2 and filled with oxygen and the anesthetic agent corresponding to the gas consumption. CO2 is removed in a CO2 absorber comprising typically NaOH or KOH soda-lime. Gases are added through fresh gas inlet, which is used to control a re-breathing circuit mixture. Another port of the circuit is connected to a ventilator controlling a circuit pressure to ventilate the patient. Through this port the ventilator provides an inspiration volume that is for a typical adult patient 500 mL into the breathing circuit during the inspiration and receives a respective volume during the expiration. The ventilator comprises a volume that preserves the expiration gas for the next inspiration. Typically this ventilator preserving volume is maximum 1500 mL.

The anesthesia delivery system where the supplied fresh gas corresponds to the patient gas consumption is called a closed circuit. These have, however, many difficulties like circuit leakages including a patient connection, a side-stream gas monitor gas sampling flow, a patient produced volatile organic compound (VOC) gases, and agent degradation reactions in the soda-lime absorber. A fresh gas flow typically somewhat exceeds the patient gas uptake to allow some margin for leakages, gas sampling without need of returning the sampling gas to the circuit, and a circuit ventilation out of the unwanted gas compounds. Even closed circuits are temporarily opened to wash out the contaminations. To prevent unwanted pressure build-up in the circuit when the fresh gas flow exceeds the patient consumption the circuit is provided also an excess gas scavenging port. This port is actively controlled to maintain the desired circuit pressure. In typical modem inhalation anesthesia the fresh gas flow rate varies from 0.5-1.5 L/min. The higher the flow is, the more open the circuit and the more anesthesia agent is lost and vice versa.

The breathing circuit volume when ventilating a normal adult patient including the patient's lung volume may be 7L. The minimal, agent preserving, fresh gas flow used to control the circuit mixture may be 0.5 L/min. With such flow the mixture concentration changes become very slow. Therefore the flow rate is often increased when the mixture composition change is needed, and reduced back to the agent preserving level afterwards. In order to affect the concentration change, multiple interventions to the machine controls are required before the minimal flow is reset for the new circuit concentration. This grabs the attention of the care personnel from the patient to the machine controls. To release the caregiver resources to the more important, automatic control of the patient concentration is desired. For this purpose, a monitoring device measures the patient concentration. A controller compares this information with a target patient concentration given by the user through a user interface and according to this comparison adjusts the delivered gas mixture in order to match the measured concentrations with the given target.

When the patient concentration is controlled automatically to match with the given target, the user attention towards the machine is reduced. In these circumstances the validity of the measured information in respect to the actual value has primary importance. All errors in a measured information will result to an error opposite in direction and equal in magnitude to the measurement error.

State of the art solution to validate the measurement information in medical feedback control loops is a duplicate monitoring e.g. a primary gas monitor is used for a feedback control and a secondary monitor is used for patient monitoring and validation. The duplication adds the system cost and complexity. Another problem of the duplicated measurement is that the two measurements may be located at different sites and hence measuring different gas resulting to false alarms. Alternatively, the respiratory monitor may be fitted to analyze periodically a known gas concentration. For this purpose, the known fresh gas concentration is periodically measured with the respiratory monitor by connecting the side-stream sampling with a valve to sample the fresh gas. An equality of the measured result with the known value then validates the measurement. This solution needs additional hardware to guide the sampling to different places. Furthermore, the fresh gas concentration may be uncertain after changing the fresh gas composition for a period of a time that depends on a fresh gas flow rate. Still further problem of the method is that during the fresh gas sampling the monitor is out of duty in measuring the patient gas. A disadvantage of all these methods adding some functionality for the safety is that these added components will reduce system reliability in providing more opportunities for failing components.

### BRIEF DESCRIPTION OF THE INVENTION

The above-mentioned shortcomings, disadvantages and problems are addressed herein which will be understood by reading and understanding the following specification.

In an embodiment, an arrangement for supervising a medical monitor includes a measurement function including at least one measurement device to make at least one measurement for obtaining a measurement information for monitoring one or more patient care signal. The arrangement for supervising the medical monitor also includes a supervision analyzer communicating with the measuring function supplying the measurement information to the supervision analyzer and which supervision analyzer is adapted to obtain a reference information created by one of the measurement function measuring patient care signal different from the measurement information and exploiting only partly same measurement components while measuring the same patient care signal as measuring the measurement information and other than the measurement function, and to compare the measurement information to the reference information to find out whether the measurement function is working properly.

In another embodiment, an arrangement for supervising a medical monitor includes a measurement function including at least one measurement device to make at least one measurement for obtaining a measurement information for monitoring one or more patient care signal. The arrangement for supervising the medical monitor also includes a supervision analyzer communicating with the measuring function supplying the measurement information to the supervision analyzer and which supervision analyzer is adapted to obtain a reference information created by one of the measurement function measuring patient care signal different from the measurement information and other than the measurement function, and to compare the measurement information to the reference information to find out whether the measurement function is working properly.

In yet another embodiment, a method for supervising a medical monitor includes activating the medical monitor to make a measurement indicative of one or more patient care signal to create a measurement information. The method for supervising the medical monitor also includes creating a reference information exploiting one of a patient care signal different from the measurement information or same patient care signal acquired using only partly same measurement components as with the measurement information or other signal than the measurement information. The method for supervising the medical monitor further includes supplying the measurement information and the reference information for comparing and comparing the measurement information to the reference information. The method for supervising the medical monitor also includes determining whether or not the measured information is acceptable and choosing one of continue as planned or take action.

Various other features, objects, and advantages of the invention will be made apparent to those skilled in art from the accompanying drawings and detailed description thereof.

### BRIEF DESCRIPTION OF THE DRAWINGS

Figure 1 is a schematic view of an arrangement for a monitor supervision; and

Figure 2 is a block diagram illustrating a method in accordance with an embodiment

### DETAILED DESCRIPTION OF THE INVENTION

Medical monitors are used to guide a patient care. A reliability of a provided information is therefore of primary importance. This becomes still emphasized when the medical monitor is connected to an automatic control system to drive a patient care in order to achieve a target level given by the clinician. Such automated control system has to involve an independent arrangement for a supervision to detect a measurement failure, issue alarms, and guide e.g. an actuator unit to a safe operating mode so that a patient safety is not compromised by a failure of the medical monitor. The supervision arrangement shall be independent of the normal control system in a way that no single failure shall corrupt the system without notice.

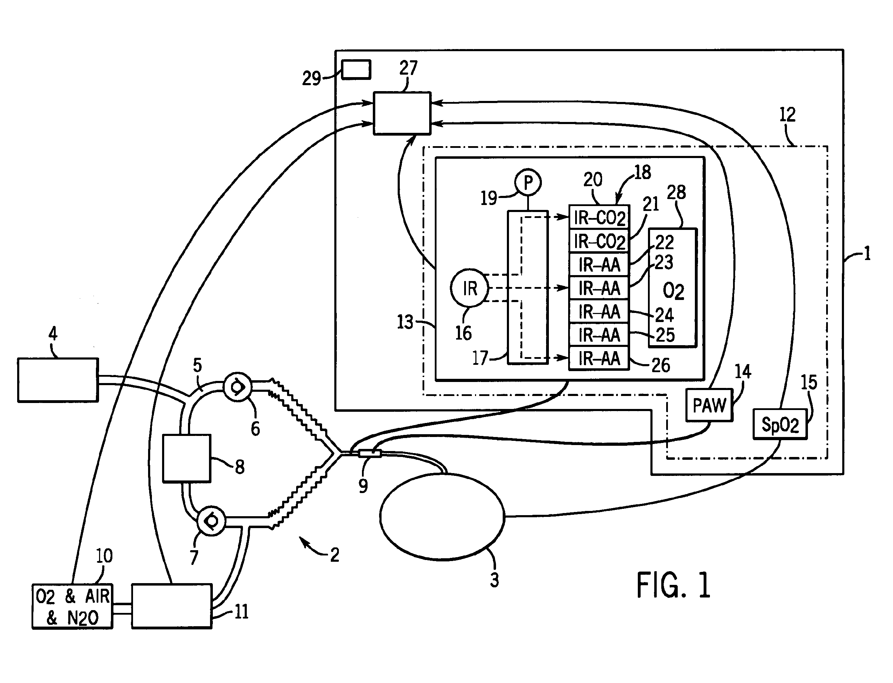

Referring to Figure 1, the medical monitor 1 is arranged to control a medical system 2 such as the actuator unit used in a medical operation and to control a patient 3. The medical system 2 in this embodiment is for anesthetizing the patient and comprises a ventilator 4 to administer a respiration gas to a patient 3 and a breathing circle 5, which is between the patient 3 and the ventilator 4 and through which the respiration gas flows. The breathing circle 5 of this embodiment is used for a re-breathing system, but could as well be modified for a non-rebreathing system. The medical system 2 comprises an exhalation valve 6 and an inhalation valve 7 both locating in the breathing circle 5 to guide exhalation and inhalation respiration gas flows and a carbon dioxide absorber 8 to absorb from the exhalation respiration gas carbon dioxide, which gas can be returned back to the patient 3. Also the medical system comprises a pressure adapter 9 located in the breathing circle for measuring a prevailing pressure of the respiration gas. The pressure adapter 7 is able to measure a pressure difference to recognize the respiration gas flow. Further the medical system 2 comprises a gas mixer 10 and a vaporizer 11. The gas mixer 10 is for feeding a fresh gas such as oxygen, air and nitrogen oxide into the breathing circle 5. A vaporizer 11 containing some anesthetic agent feeds vaporized agent into the breathing circle 5 to keep the patient 3 in a desired anesthetic level.

In order to control that nothing goes wrong while anesthetizing the patient various parameters must be monitored by the medical monitor 1 comprising a measurement function 12 which may include one or more measurement devices 13, 14, 15 depending on the need.

The respiration gas of the patient 3 is continuously controlled by measuring some specific agents like oxygen, carbon dioxide and/or anesthetic agent. This is made by the first measurement device 13 such as a gas analyzer shown in Figure 1, which typically comprises various components 16, 17, 18. The component 16 is for an infrared radiation, such as an infrared radiator, the component 17 is a gas volume such as a gas chamber, the component 18 is for a detector unit. Also a pressure sensor 19 is useful. Such IR analyzer can be used to measure anesthesia gases and CO2. The first measurement device 13 may be either a side stream type as shown in Figure 1, but also a main stream type. The side stream one draws samples from the breathing circle 5 to make measurements, but the main stream one is directly assembled in the breathing circle 5 and is able to measure the gas flowing in the breathing circle 2. Irrespective of whether the first measurement device 13 is the side stream type or the main stream type an electromagnetic radiation emitted by the component 16 transmits the component 17 for the gas volume containing the respiration gas, and which transmitted radiation that has been damped, is received by the component 18 for the detector unit for analyzing purposes. It should be understood that the component 17 for the gas volume in the main stream alternative is same as a respiration gas flow channel. The component 18 for the detector unit may include several components 20-26 for detections such as detectors adjacent to each other with suitable filters (not shown) providing sensitivity to specified radiation wavelength. According to the embodiment there are at least two components for the detection providing a measurement result for each analyzed gas. A pressure inside the gas chamber is measured by the pressure sensor 19. The first measuring device 13 may further comprise an oxygen analyzer 28, which may be based on a paramagnetic phenomenon to measure an oxygen content of the respiration gas. Alternatively, 02 can me measured using a chemical or fuel cell analyzer. Typical to those is a relatively short life cycle after which the sensor (not shown) must be replaced. To identify the need for the sensor renewal a measurement chamber for 02 sensor can be equipped with duplicate pair of electrodes and electrolyte.

The second measurement device 14 such as a pressure sensor is arranged to measure a pressure of the breathing gas inside the breathing circle 5. The second measurement device 14 can be a separate part connectable by means of a tube to the pressure adapter 9 of the breathing circle 2 or integrated with the pressure adapter 9 into one single piece.

Very often a tissue of the patient is monitored non-invasively by the third measurement device 15 such as a pulse oximeter. The tissue such as a blood is monitored and especially an oxygenation level of the blood and/or a pulse rate is measured with a sensor (not shown) connectable about an extremity of the patient 3 such as a finger or toe. The sensor comprises an emitter emitting a light and a detector receiving a light transmitted by the tissue of the patient 3. This is a transmittance type sensor, but as well a reflective type sensor can be used where the detector receives a light reflected by the tissue. Naturally a holder is needed to position the sensor connected to the third measurement device 15 making necessary calculations based on the measured values of the sensor. The sensor and the third measurement device 15 can be integrated into one single piece or can be separate and connectable by a wire or wireless. The third measurement device 15 with a specific sensor can be used to measure whatever parameter desired in the tissue of the patient 3.

The medical monitor 1 also comprises a supervision analyzer 27 arranged to supervise the measurement function 12, because it is important to know that it is working properly and make sure a patient safety. The supervision analyzer may also be separate from the medical monitor 1. An alarm/display unit 29 connected to the supervision analyzer 27 will take care of an audio alarm and/or a visual alarm in case one of the first measurement device 13, the second measurement device 14 or the third measurement device 15 or only part of one of these devices is out of order. The supervision analyzer 27 can guide the measurement function 12 or one of the first measurement device 13, the second measurement device 14 or the third measurement device 15 to change their operation or to limit their operation or to disconnect either the whole measurement function 12 or one of the first measurement device 13, the second measurement device 14 or the third measurement device 15.

The supervision analyzer 27 is adapted to monitor besides the measurement information is reliable but also desired connections exist. Therefore the supervision analyzer 27 is wire- or wireless connectable to the measurement function 12 including the first measurement device 13, the second measurement device 14 and the third measurement device 15 to get a measurement information and also in some cases the reference information. Further especially according to Figure 1 embodiment it is advantageous to connect the supervision analyzer 27 to the medical system 2 including the vaporizer 11 in order to get a reference information about these functions. The reference information and the measurement information makes possible to compare these information with each other and to take measures in case this compared information taking into account a comparison acceptability limit gives any reason to do that. The comparison acceptability limit may have been fed into the supervision analyzer already at a factory or it may happen afterwards. The comparison acceptability limit may be equal in which case the supervision analyzer interpretes the reference information and the measurement information should be equal. Besides the equality comparison the comparison acceptability limit may be a limit value or a range of limits.

If the comparison result is not according to the comparison acceptability limit the supervision analyzer 27 may choose to take an action including the alarm and that may include a change of the operation mode, but if everything proves to be correct then the supervision analyzer 27 may choose to continue as planned without an intervention. It is possible to have the measurement information from the measurement function 12 and the reference information from the medical system 2, but as well both the reference information and the measurement information can be from the measurement function 12. If they are from the same measurement function 12 it is advantageous to have them from different measurement devices 13, 14 or 15. Further both the measurement information and the reference information can be besides from the same measurement function 12, but also from the same first measurement device 12 whereby one of the components 16, 17, 18, 20, 21, 22, 23, 24, 25, 26 used while determining the measurement information and reference information is different and other components can be the same.

Thus according to one embodiment the supervision analyzer is arranged to supervise an operation of the measurement function 12 and connections to the breathing circle 2 by comparing the measurement information from at least one of its independently operating first measurement device 13, second measurement device 14 and third measurement device 15 with a reference information from one of its independently operating first measurement device 13, second measurement device 14 and third measurement device 15 or comparing at least the measurement information from one of its independently operating first measurement device 13, second measurement device 14 and third measurement device 15 with the reference information received from the medical system 2, and identifies those which are out of function or are not connected properly to the breathing circle 5.

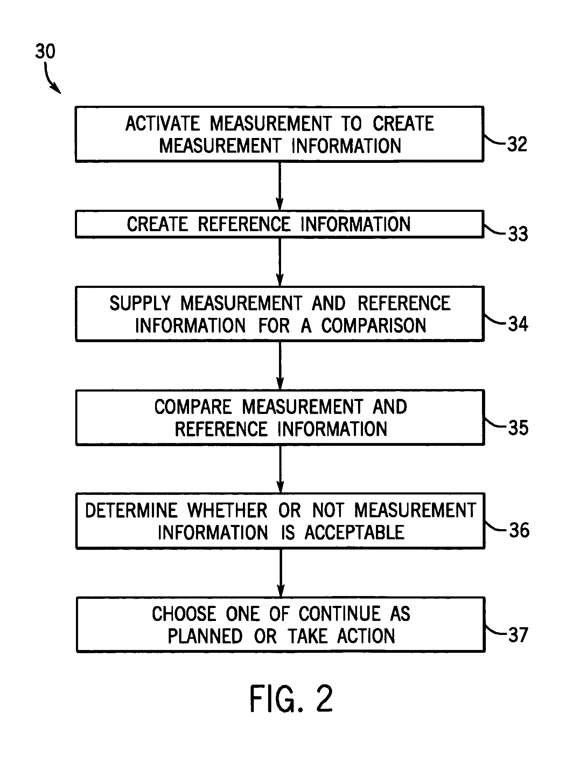

The supervision analyzer 27 and its measurement function 12 and other features of Figure 1 will also be introduced with a method 30 to supervise the measurement function 12 as shown in Figure 2. It is important to note that there are also many other possible and useful supervisory methods included in the method 30.

Step 32 is to activate a measurement by means of the measurement function 12 and to create the measurement information. The measurement function 12 for monitoring one or more patient care signal can be based on any useful method known in the art. The measurement can be made at least by one of its independently operating first measurement device 13, second measurement device 14 and third measurement device 15. The first measurement device 13 is adapted to emit an infrared radiation through a sample flowing through the component 17 for the gas volume and which transmitted radiation is received by the component 18 for the detector unit having one component 20 for carbon dioxide detection, and components 22-26 for identification of the anesthetic agent component, and at least one of the components 22, 23, 24, 25, 26 for the measurement of identified anesthetic agent for defining a concentration. The number of the components for the detection in this kind of gas analyzer depends on the number of gas components identified, because measurement wavelengths are different. Also the first measurement device 13 includes the oxygen analyzer 28, which is adapted to measure oxygen concentration of the respiratory gas. The second measurement device 14 is adapted to measure the respiration gas pressure and/or pressure difference of the breathing circle 5.

At step 33 the reference information is created. This can be created by making a measurement by means of the measurement function 12. A measurement method for different patient care signal and differing from the measurement method of step 32 is typically used as the reference information, but in case the reference information is from the measurement function 12 using an equivalent measurement method for same patient care signal while obtaining the measurement information of step 32 these measurements should exploit only partly same or in other words at least partly different measurement components 20, 21, 22, 23, 24, 25, 26. The reference information can be created using some other than measurement information obtained from the measurement function 12 such as a predetermined signal from the medical system 2. Examples of the reference information are as follows:
- The third measurement device 15 is adapted to measure the patient hemoglobin oxygen saturation of the patient tissue, very often called Sp02.
- A capnograms can be collected by means of the first measurement device 13 emitting infrared radiation through a gas sample to the component 20 for carbon dioxide detection and an oxygram can be collected by means of the oxygen analyzer 28 both grams showing characteristic inspiration and expiration phases.
- To use equivalent measurement methods with a different component 21 for the detection of carbon dioxide of the respiration gas and/or other components 22, 23, 24, 25, 26 than those ones used in step 32 for measured anesthetic agent concentration of the respiration gas.
- A sample gas pressure measurement is made by the pressure sensor 19 of the first measurement device 13.
- Respiration gas pressure waveform of the breathing circle 5 with characteristic inspiration and expiration phases is collected.
- The predetermined signal can be created by the vaporizer 11 having an information of an anesthetic agent type.

The supervision analyzer 27 receives at step 34 both the measurement information from the measurement function 12 of the medical apparatus 1 such as the respiration gas component concentration and/or the identification of the component, the oxygen content of the respiration gas, the respiration gas pressure, the pressure difference and the reference information such as the patient hemoglobin oxygen saturation, the grams for CO2 and 02, the respiration gas components created at step 33, the sample gas pressure and the collected respiration gas pressure waveform of the breathing circle 5. This measurement information can be used by the supervision analyzer 27 while determining whether or not the measurement function is working properly. Also this comparison may reveal whether one or more connections to the breathing circuit 2 are active.

The supervision analyzer 27 compares at step 35 the measurement information and the reference information. Examples:
- The measured respiration gas component concentrations of step 32 are compared to measured gas component concentrations of step 33 when components 20, 21, 22, 23, 24, 25, 26 for the detections are different, but measurement methods are equivalent.
- The identification of the measured respiration anesthesia gas component can be compared to the anesthetic agent type information from the vaporizer 11.
- The respiration gas pressure of the breathing circle 5 measured by the second measurement device 14 is compared to the sample gas pressure measurement made by the pressure sensor 19 of the first measurement device 13.
- The measured oxygen content of the respiratory gas can be compared to the patient hemoglobin oxygen saturation of the patient tissue.
- The measured oxygen breath cycle variation can be compared to CO2 capnogram with characteristic inspiration and expiration phases, too.
- The measured CO2 concentration breath cycle variation is compared with 02 oxygram variation with characteristic inspiration and expiration phases
- The measured respiration gas pressure difference is compared with the collected respiration gas pressure waveform and CO2 capnogram.
- The measured respiration gas pressure with normal inspiration and expiration phases is compared with the collected sample gas pressure with normal inspiration and expiration phases.

At step 36 the supervision analyzer 27 determines based on the comparison and the comparison acceptability limit whether or not the measured information is acceptable. As an example:
- To determine whether or not the comparison result between the measured respiration gas component concentration of step 32 and the measured gas component concentration of step 33 is according to the comparison acceptability limit.
- The comparison made between the measured respiration anesthetic gas component and the anesthetic agent type information is compared to the comparison acceptability limit which in this case can be either equal or not equal.
- The supervision analyzer 27 determines whether the comparison between the respiration gas pressure of the breathing circle 5 and the sample gas pressure measurement is acceptable according to the comparison acceptability limit.
- To determine whether the comparison made between the measured oxygen concentration of the respiration gas and the measured patient hemoglobin oxygen saturation of the patient is according to the comparison acceptability limit.
- The comparison made between the measured O2 oxygram and CO2 capnogram is compared with the comparison acceptability limit. The comparison made between the measured CO2 concentration and 02 oxygram is compared to the comparison acceptability limit.
- The comparison made between the measured respiration gas pressure difference and the collected respiration gas pressure waveform and CO2 capnogram is compared to the comparison acceptability limit.
- The comparison made between the measured respiration gas pressure and the collected sample gas pressure is compared to the comparison acceptability limit.

Based on the determination the supervision analyzer 27 may choose one of continue as planned or take an action at step 37. The choice can be to continue as planned in case the determination made at step 36 proves that the measurement information was acceptable. Instead if the determination made by supervision analyzer 27 proves the comparison between the measurement information and the reference information is not according to the comparison acceptability limit the supervision analyzer 27 takes at step 37 a predetermined action including giving an alarm signal. The supervision analyzer may also change the operation mode of the anesthesia system. Following examples clarifies this step:
- If the determination made by the supervision analyzer proves that the comparison result between the measured respiration gas component concentration of step 32 and the measured gas component concentration of step 33 is not according to the comparison acceptability limit then the measurement information of step 32 is not reliable requiring the supervision analyzer 27 to take a predetermined action. If it had been acceptable gas measurements could continue as planned which may mean unchanged or unaffected.
- If the measured respiration gas component and the anesthetic agent type information do not prove that both the component and the anesthetic agent type are equivalent then the supervision analyzer takes a predetermined action and e.g. gives an alarm for the user or interrupts the anesthetic agent's feed. Otherwise if identical operation remains unchanged or unaffected.
- If the determination proves that the blood oxygen content of the patient tissue and the measured oxygen content of the respiratory gas are conflicting taking into account the comparison acceptability limit then the measured oxygen content of the respiratory gas is not reliable in which case there is a reason to take an action.
- If the measured 02 oxygram is stable or rather stable, but CO2 capnogram includes characteristic inspiration and expiration phases, it can be determined after comparing to the comparison acceptability limit that the oxygen analyzer 28 is out of function and not reliable.
- In case the measured CO2 capnogram is stable or rather stable but 02 oxygram includes characteristic expiration and inspiration phases it can be determined after comparing to the comparison acceptability limit that especially the component 20 for carbon dioxide detection and possibly the whole first measurement device 13 perhaps excluding the oxygen analyzer 28 is out of function and in which case there is a reason to take an action.
- If the second measurement device 14 for the pressure difference measurement of the respiratory gas is out of the function when compared to the comparison acceptability limit in case the measured respiration gas pressure difference is stable or rather stable, but the respiration gas pressure waveform and CO2 capnogram includes characteristic inspiration and expiration phases. If not working then it is necessary to take an action.
- If the measured respiration gas pressure is stable but normal inspiration and expiration phase are detectable in the gas chamber 17 pressure measured by the pressure sensor 19, this may prove after comparing to the comparison acceptability limit that the second measurement device 14 for the respiration gas pressure measurement is out of function.

Hereinabove described method may provide redundant safety by improving the self-diagnostics and self-supervision in the medical monitor 2 such as the respiratory gas monitor. Such self-supervision may take the advantage of the existing system components to identify all control device errors that might risk the patient safety. Also the self-supervision is accomplished by taking an advantage of the known interdependencies between the parameters measured by the medical monitor 1 in the medical system 2. The advantage is that in many cases existing medical systems 2 already includes most components needed with the method. The most important thing of the embodiment is that a patient safety may be increased.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. An arrangement for supervising a medical monitor (1) comprising:
a measurement function (12) including at least one measurement device (13,14,15) to make at least one measurement for obtaining a measurement information for monitoring one or more patient care signal; and
a supervision analyzer (27) communicating with said measuring function (12) supplying said measurement information to said supervision analyzer (27) and which supervision analyzer (27) is adapted to obtain a reference information created by one of said measurement function (12) measuring patient care signal different from said measurement information and exploiting only partly same measurement components (20, 21, 22, 23, 24, 25, 26) while measuring the same patient care signal as measuring said measurement information and other than said measurement function, and to compare said measurement information to said reference information to find out whether said measurement function (12) is working properly.

2. The arrangement according to claim 1, wherein said at least one measurement device is one of a first measurement device (13), a second measurement device (14) and a third measurement device (15) for monitoring one or more patient care signal of the patient (3).

3. The arrangement according to claim 2, wherein said first measurement device (13) is adapted to make a gas measurement of a respiration gas of the patient, which gas measurement includes at least one of identification of a gas component or analysis of a concentration of a gas component.

4. The arrangement according to claim 2, wherein said second measurement device (14) is adapted to measure a pressure of a respiration gas.

5. The arrangement according to claim 2, wherein said third measurement device (15) is adapted to monitor a tissue of the patient (3) such as an oxygenation level of a blood and/or a pulse rate.

6. The arrangement according to claim 1, wherein said reference information was created exploiting only partly same measurement components (16, 17, 18, 20, 21, 22, 23, 24, 25,26) such as a component (16) for an infrared radiation and a component (17) for a gas volume as used with equivalent measurement for obtaining said measurement information, but using a different component for a detection.

7. The arrangement according to claim 1, wherein said measurement information is obtained from one measurement device (13,14,15) and said reference information is created by other than the measurement device used for said measurement information.

8. The arrangement according to claim 1, wherein said reference information created by other than said measurement function is from a vaporizer (11) indicating an anesthetic agent type.

9. The arrangement according to claim 1, further comprising an alarm/display unit (29) for an alarm signal in case said measurement function (27) is out of order.

10. A method for supervising a medical monitor (1) comprising:
activating (32) said medical monitor to make a measurement indicative of one or more patient care signal to create a measurement information;
creating (33) a reference information exploiting one of a patient care signal different from said measurement information or same patient care signal acquired using only partly same measurement components as with said measurement information or other signal than said measurement information;
supplying (34) said measurement information and said reference information for comparing;
comparing (35) said measurement information to said reference information;
determining (36) whether or not the measured information is acceptable; and
choosing (37) one of continue as planned or take action.

11. The method according to claim 10, wherein determining (36) is adapted to take into account a comparison acceptability limit.

12. The method according to claim 10, wherein determining (36) is based on said comparing (35) and a comparison acceptability limit.

13. The method according to claim 10, wherein choosing (37) to continue as planned includes to remain a monitor operation unchanged.

14. The method according to claim 10, wherein choosing (37) to take action includes giving an alarm signal.

15. The method according to claim 10, wherein activating (32) said medical monitor to make a measurement includes one of a gas measurement of a respiration gas of the patient, which gas measurement includes at least one of identification of a gas component or analysis of a concentration of a gas component, or a pressure measurement of a respiration gas, or a tissue monitoring of the patient (3) such as an oxygenation level of a blood and/or a pulse rate.
